# NEW EUROPEAN PATENT SPECIFICATION

(11) **EP 0 527 199 B2**
(45) Date of publication and mention of the opposition decision: **30.08.2000**
(45) Mention of the grant of the patent: 24.07.1996
(21) Application number: 91909565.3
(22) Date of filing: 01.05.1991
(51) Int. Cl.: C12Q 1/68

(54) **Compositions for use in a method for treating a human suffering from multiple sclerosis**
Stoffgemische zur Anwendung in einem Verfahren zur Behandlung eines an multipler Sklerose leidenden Menschen
Compositions pour la mise en oeuvre d'une méthode pour le traitement d'un patient souffrant de sclérose en plaques

(30) Priority: 01.05.1990 US 517245
(43) Date of publication of application: 17.02.1993
(60) Divisional application: 96100852.1
(73) Proprietor: THE BOARD OF TRUSTEES OF THE LELAND STANFORD JUNIOR UNIVERSITY, Stanford California 94305 (US)
(72) Inventor: STEINMAN, Lawrence, Palo Alto, CA 94301 (US); OKSENBERG, Jorge, Palo Alto, CA 94306 (US); BERNARD, Claude, North Ballwin, VIC 3083 (AU)
(74) Representative: Kraus, Walter, Dr.
(86) International application number: US9102991
(87) International publication number: WO9117268

(56) References cited:
- EP-A- 0 203 403
- EP-A- 0 340 109
- WO-A-86/06413
- WO-A-88/00209
- WO-A-88/10314
- WO-A-89/04875
- WO-A-91/01133
- WO-A-91/09623
- WO-A-91/15225
- WO-A-91/91133
- US-A- 4 874 845
- US-A- 4 886 743
- US-A- 5 126 132
- JOURNAL OF THE NATIONAL CANCER INSTITUTE vol. 80, no. 13, 7 September 1988, BETHESDA, MD, USA pages 1016 - 1026 C.L. SLINGLUFF ET AL.
- DIABETES vol. 39, January 1990, ALEXANDRI,VA,USA pages 96 - 103 H.A. ERLICH ET AL. 'HLA-DQbeta sequence polymorphism and genetic susceptibility to IDDM'
- SCIENCE vol. 249, 10 August 1990, LANCASTER, PA US pages 672 - 674 T. NITTA ET AL. 'Predominant expression of T-cell receptor Valpha7 in tumor-infiltrating lymphocytes of uveal melanoma'
- Proceedings of the National Academy of Sciences (US), Vol. 86, issued February 1989, J.R. OKSENBERGE et al; pp. 988-992;
- The Journal of Immunology, Vol. 136, No. 9, issued 01 May 1986, L.A. MATIS et al., "Adoptive Immunotherapy of a Syngeneic Murine Leukemia with a Tumor- Specific Cytotoxic T Cell Clone and Recombinant Human Interleukin 2: Correlation with Clonal IL2 Receptor Expression", pages 3496-3501; see entire document.
- Journal of Immunogenetics (Oxford), Vol. 16, No. 6, issued 1989, Y. FUKUI et al., "Glycosuria and Insulitis in NOD Mice Expressing the HLA-DQW6 Molecule", Abstract.
- Bull. Soc. Ophtalmo., 90(8-9), 1990, p.761-4
- Nervenarzt, 56(8), 1985, p.442-8
- Ann. Rev.Immunol.,5, 1987, 585-620
- Journal of Neuroscience Research, 28, 1991, 280-90
- Nature, 341, 12 October 1989, 541-544
- Nature, 345, 24 May 1990, 344-6
- Nature, 362, 4 March 1993, 68-70
- Proc. Natl. Acad. Sci. USA, 86, November 1989, 8941-5
- Science, 246, 1989, 668-70

## Description

The invention relates to a composition for use in a method for treating a human being suffering from multiple sclerosis.

Autoimmune diseases are a result of a failure of the immune system to avoid recognition of self. The attack by the immune system of host cells can result in a large number of disorders, including such neural diseases as multiple sclerosis and myasthenia gravis diseases of the joints, such as rheumatoid arthritis, attacks on nucleic acids, as observed with systemic lupus erythematosus and such other diseases associated with various organs, as psoriasis, juvenile onset diabetes, Sjogren's disease, and thyroid disease. These diseases can have a variety of symptoms, which can vary from minor and irritating to life-threatening.

Despite the extensive research efforts that have been involved with elucidating the basis for these diseases, the diseases for the most part have been recalcitrant to an understanding of their etiology in the development of therapeutic modes. Many of the diseases are believed to be associated with lymphocytic involvement, which can result in attack and degradation of proteins, cytotoxicity, and the like.

In the case of cancer, tumor infiltrating lymphocytes (TIL) are believed to be part of the body's defense mechanism to destroy the tumor. Efforts have been made to expand T-cells found in tumor tissue and return the culture expanded cells to the host.

The complexity of the immune system has been a daunting barrier to an understanding of the autoimmune diseases and the immune response to neoproliferative diseases. In attempting to understand the mechanisms involved with the immunological response, there is substantial interest in understanding in what manner the system degenerates to attack self. By understanding the relationships between the components of the immune system, the manner in which the immune system distinguishes between self and non-self, and the components the immune system associated with a particular disease, ways may be developed to diagnose individuals who may be susceptible to autoimmune diseases and provide therapies to protect such susceptible individuals from autoimmune disease during its onset and during its progress or to treat individuals with specific T-cells.

Multiple sclerosis (MS) is an inflammatory disease of the central nervous system characterized by myelin destruction (McFarlin and McFarland, New Engl. J. Med. 307, 1183-1251 (1982)). At the site of demyelination, depletion of oligodendroglia cells and proliferation of astrocytes is usually observed. Raine and Traugatt, Immunoregulatory Processes in Experimental Allergic Encephalomyelitis and Multiple Sclerosis, Elsevier, New York, (1984) 151-212; Prineas and Wright, Lab. Invest 38, (1978) 409-421. There is an accumulation of morphologically identifiable macrophages, plasma cells and T lymphocytes, characteristic of an inflammatory response in the brain. Prineas, Handbook of Clinical Neurology, 3, Elsevier, New York, (1985) pp. 213-257. MHC Class II, positive antigen presenting cells and activated T-cells secreting various cytokines are present. Woodroofe et al., J. Neurol. Sci. (1986) 74, 135-152; Hafler and Weiner, Ann. Neurol. (1987) 22, 89-93; Hafler and Weiner, Immunol. Rev. (1987) 100, 307-332; Hoffman, J. Exp. Med. (1989) 170, 607-612. Several lines of evidence suggest that T lymphocytes migrate from the peripheral blood through the CNS compartment and participate directly in the promotion of brain lesions. Hoffman et al., J. Immunol. (1966) 136, 3239-3245; Traugott, J. Neuroimmunol. (1985) 4, 201-221. In studies of MS plaque tissue with monoclonal antibodies, it has been shown that the majority of T-cells have the helper inducer CD4 positive phenotype. Sobel et al., J. Exp. Med. 167, (1988) 1313-1322. Also, by restriction fragment length polymorphism analysis, T-cell receptor Vα and Vβ genes have been shown to contribute to the genetic control of susceptibility to this disease. Beall et al., J. Neuroimmunol. (1989) 21, 59-66; Seboun et al., Cell 57, (1989) 1095-1100; Oksenberg et al., Proc. Natl. Acad. Sci. USA (1989) 86, 988-992; Barth et al., J. Immunol. (1990) 144, 1531 describe the use of TIL cells in the treatment of tumors.

HLA-DR2Dw2 is associated with increased susceptibility to MS. Terasaki et al. (1976) Science 193:1245-1247. Susceptibility to MS has been associated with certain MHC Class II genes. Oksenberg and Steinman, Current Opinion in Immunology (1990) 2:619-621. At the cellular level, oligoclonality of T-cells has been described in the cerebrospinal fluid (CSF) of MS patients. Lee et al., Ann. Neurol. (1991) 29:33-40. Oksenberg et al., Nature (1990) 345:344-346 describes the use of PCR to amplify TCR Vα sequences from transcripts derived from MS brain lesions. Wucherpfennig et al. Science (1990) 248:1016-1019 and Ota et al., Nature (1990) 346:183 report studies of T-cell clones in man that recognize myelin basic protein.

EP-A-0 340 109 relates to the characterisation of some aspects of the correlation between T-cell receptors and autoimmune diseases. There is no correlation for the specific TCR V region genes as disclosed in the present application.

Tissue from autoimmune or neoplastic lesions is assayed for the presence of T-cells and the T-cell receptor variable regions. The presence of clonal or oligoclonal variable regions of the T-cell receptor indicates that-these variable regions are associated with the autoimmune disease or response to neoplastic tissue. Various therapies may be employed prophylactically or therapeutically to inhibit the autoimmune effect of the T-cells on the target cell or protein or enhance the response in relation to neoproliferative tissue.

Specific MHC phenotypes may be associated with susceptibility to autoimmune diseases and specific T-cell clones associated with the disease. Therapies may be employed which inhibit T-cell activity against target proteins associated with the disease.

It is demonstrated that the variable rearrangements of T-cells associated with specific sites of autoimmune disease, have a restricted repertoire, so that a relatively small number of T-cell variable regions of both the α and β subunits are observed. Furthermore, a limited repertoire of MHC type is associated with the disease and as to these repertoires, there will generally be a restricted repertoire of T-cell receptor variable regions.

In particular, with MS patients it is found, particularly in plaques in the brain associated with the disease, that T-cells are isolated which have rearranged germline DNA to provide for expression of the T-cell receptor. This may be contrasted with brains of normal healthy individuals, where the T-cells found in the brain have unrearranged germline DNA.

By identifying a particular Class II HLA haplotype or molecular phenotype, one can than identify particular Vα and Vβ variable regions associated with multiple sclerosis. Once the autoimmune associated T-cell receptor variable regions are identified, one may then use the composition of the invention in the various therapies which are described in the subject application for prophylaxis or treatment.

Of interest are specific V regions of the α subunits of the T-cell receptor.

Accordingly the present invention relates to a composition for use in a method for treating a human being suffering from multiple sclerosis as a result of attack by T-cells of native tissue, said composition comprising an antibody specific for a variable region of the α subunit of a human T-cell receptor, said region belonging to one of the V_{α} families 8-10, 12 or 16, or combinations thereof and being capable of inhibiting the activity of T-cells having said T-cell receptor or subunit thereof.

Methods and compositions are provided for determining T-cell receptor variable regions related to multiple sclerosis. Also specific MHC profiles may be identified associated with multiple sclerosis, which will provide for monitoring of such patients to identify initiation of symptoms of said disease.

For multiple sclerosis, by identifying specific T-cell receptor (TcR) variable regions associated with the disease, therapies may be employed to inhibit the attack of the T-cells having such variable regions on the target cells or proteins. The therapies may involve ablation of T-cells carrying the particular variable regions, administration of compounds which inhibit binding of the T-cell receptor to the target cell, or prevention of the degenerative effects of the binding of the T-cell to the target cell or protein.

The T-cell receptor has two subunits involved in binding, either α and β, or γ and δ. The variable regions associated with the subunits have a similar organization to those of the immunoglobulins, the β and γ subunits having a variable region which comprises exons associated with the V, D and J regions, while the α and δ subunits comprise exons associated with the V and J regions. By rearrangement of germline DNA, the exons are joined to the constant or conserved region and by subsequent splicing of the messenger RNA, an open reading frame is achieved which encodes the subunit. Depending upon the particular genetic inheritance of the host, the spectrum of the variable region loci of an individual will be different from most other individuals. In addition, not all variable region exons present may rearrange to form a functional T-cell receptor subunit. By determining the loci which are rearranged to form functional variable regions, which variable regions are associated with autoimmune lesions, one can diagnose multiple sclerosis establish the existence of a chronic episode, and treat the disease, prophylactically or therapeutically, by inhibiting the degenerative effect of the T-cells.

T-cell receptors may be divided into two categories: the CD4 helper-inducer T-cell receptors, which T-cell receptors bind to Class II MHC; and the CD8 suppressor-cytotoxic T-cell receptors, which T-cell receptors bind to Class I MHC. For the most part, the T-cells associated with such diseases as multiple sclerosis are the CD4 positive phenotype, though CD8 can also play a role.

The variable (V) loci by themselves or in conjunction with the J loci of the T-cell receptors of T-cells found at the site of the lesion may be identified in a number of different ways. Particularly, the lesion or plaque is isolated and total RNA, from which cDNA may be prepared, or DNA is prepared according to standard procedures. To provide for more accurate results, the cDNA is amplified by any convenient technique, such as the polymerase chain reaction (PCR), cloning, or the like. In the case of the polymerase chain reaction, primers are employed which will identify the particular variable region which either has been expressed as identified by cDNA, or has been rearranged, so as to be associated with a J and C region.

Primers may be selected in accordance with the known sequences of conserved regions of the T-cell receptor subunits. It is found, that there will usually be 1 and no more than about 7, usually not more than about 5, generally only about 3 common, variable regions of the T-cell receptor subunits associated with the lesions. Therefore, with neural disorders, where the tissue is available from a deceased or from a biopsy having the particular disorder, one may relate the presence of T-cells in the lesions to the disease and, further, relate the particular V regions and J regions associated with the T-cells present in the lesion with the MHC antigen type of the deceased. In this manner, one may provide for identification of the variable regions of humans suffering from the disease with the MHC type and be able to treat the disease accordingly. With genomic DNA, one would establish the presence of the rearrangement in the DNA by having primers which relate to substantially conserved regions of the variable region and the joining of the constant region, either coding or non-coding regions. Alternatively, by cloning, one could sequence the DNA and establish the identity of the variable region. Isolation of mRNA from the tissue in question, reverse transcription to cDNA and then amplification and identification of the rearranged V-C product will also define the disease related marker.

Instead of the nucleic acid as the basis for the diagnosis, by having a battery of monoclonal antibodies, various techniques may be employed for identifying the binding of the antibody to the T-cells. Thus, flow cytometry, e.g. a FACS scan, may be employed, where the antibodies are labeled with a fluorescer and those T-cells to which the antibodies bind would then identify the particular variable region.

A 100% correlation is normally not to be expected, nor will it be necessarily achieved. It will usually be satisfactory that in at least 60%, preferably 70%, of the hosts positive for the disease, the variable region locus associated with the disease is present in host T-cells, particularly in rearranged form. Similarly, in fewer than about 50%, preferably in fewer than about 30% of the hosts which do not present the symptoms of the disease, the rearranged variable region is absent. These percentages should be based upon a statistically significant number of hosts.

For multiple sclerosis, brain plaques or cerebrospinal fluid may be employed as a source of the T-cells.

Once the rearranged variable region(s) are established, one may then identify T-cell variable region allele(s) or T-cell receptors in a host, in association with the HLA or MHC restriction, as indicative of the propensity for the disease or the existence of the disease or the neoproliferative cell responsive T-cells. Where it appears that the disease is associated with one or a plurality of T-cells, the presence of one or more of the T-cell receptors having the rearranged variable regions associated with the disease will indicate the greater or lesser likelihood for the occurrence of the disease.

For diagnosis of multiple sclerosis, either the nucleic acid or antigen may be detected. For nucleic acid detection, DNA or RNA in cells may be isolated by any convenient means and by employing appropriate probes, in conjunction with techniques, such as Southern transfer, dot-blots, or the like, the presence of the rearranged V region may be detected. Depending upon the nature of the disease, there may be an opportunity for prophylactic intervention to reduce the potential for the disease occurring.

If one wished to determine the number of cells which are expressing the T-cell receptors associated with the disease, this can be achieved in a number of ways. The messenger RNA may be isolated from T-cells and probed with an appropriate probe for the V gene region. By employing Northern techniques, one can detect the presence of the messenger encoding the T-cell receptor and obtain a qualitative value for the amount of T-cell receptor being expressed containing the particular V region gene. Alternatively, one may prepare cDNA from the messenger and using the polymerase chain reaction, amplify the amount of messenger and determine the number of T-cells expressing the particular variable region in this manner.

More conveniently, one may use antibodies as described previously which are specific for the V region and/or the J region alleles or potentially the combination V-J for the α subunit. In this way, one may detect the V region and the J region, With the β subunit, intervention of the D region makes it unlikely to find a antibody which would be specific for the VDJ sequence, but the V and/or J region may be detected individually.

Antibodies may be prepared in accordance with conventional ways, particularly employing the monoclonal antibody techniques as described, for example in U.S. Patent Nos. 4,690,893; 4,713,325; 4,714,681; 4,716,111; and 4,720,459.

Any of a number of techniques may be employed for identifying the presence of a T-cell receptor binding to the particular monoclonal antibody or antiserum. A wide variety of labels have been used for detection, such as particles, enzymes, chromophores, fluorophores, chemiluminescence, and the like. Any particular label or technique which is employed is not critical to this invention and any convenient technique may be employed. The techniques may be either competitive or non-competitive methodologies, including sandwich methodologies. The cells will usually be lysed to provide membrane-free proteins in accordance with conventional techniques. Cellular debris may be removed and the protein extracted and harvested. Alternatively, intact cells may be employed and detected by fluorescence activated cell sorting or the like.

For therapeutic purposes, there may be an interest in using human antibodies. Normally, one will not be permitted to immunize a human host with the T-cell receptor or fragment thereof to activate T-cells specific for the sequence of interest. However, there are alternatives, in that mice or other lower mammals may be immunized, and the genes encoding the variable regions of the antibodies specific for the T-cell region of interest isolated and manipulated by joining to an appropriate human constant region, and optionally, the complementary determining regions (CDR) used to replace the CDRs of a human antibody by genetic engineering. The resulting chimeric construct, comprising a lower mammal variable region or CDRs and a human constant region may then be transformed into a microorganism or mammalian host cell in culture, particularly a lymphocyte, and the hybrid antibodies expressed. Of particular interest would be IgG constant regions. See, for example, EP-A-0 173 494. Also recent techniques suggest random association of immunoglobulin genes from a human host for expression in a non-human cell host e.g. prokaryotic, and screening for affinity.

In some instances, it may be satisfactory to use mouse antibodies, where tolerance can be achieved or some degree of immune suppression may be involved. Immune suppression may be achieved with cyclosporin, irradiation, anti-leu3 (anti-CD4) (U.S. Patent No. 4,681,760), or the like.

The antibodies may be used in a variety of ways, for example, for inhibiting binding between the T-cell and the target cell, for killing of T-cells, or for isolating the T-cells. In the first situation, the entire antibody may be administered, or Fab fragments, or even only the Fv region. By removing all or a portion of the constant region, there may be a reduction in the immune response. For selectively killing the T-cells carrying the particular V region, one may use a variety of immunotoxins, which may include the antibody or specific binding fragment thereof, bonded to all or a portion of a plant toxin, such as ricin, abrin, etc., or diphtheria toxin. By employing an appropriate antibody isotype, e.g., IgM or IgG₃, the complement cascade may be enlisted. Alternatively, a radioactive substituent may be used which provides for a lethal dosage upon binding of the antibody to the host cell. Another choice is to use an antibody or fragment thereof conjugated with a cytolytic agent for specific elimination of the undesired T-cells. Finally, the T-cell can be removed by extracorporeal means, such as plasmapheresis, where the plasma may be passed through or over antibodies bound to a support, with the undesired T-cells being selectively removed.

For therapeutic purposes, the antibody may be formulated with conventional pharmaceutically or pharmacologically acceptable vehicles for administration, conveniently by injection. Vehicles include deionized water, saline, phosphate-buffered saline, Ringer's solution, dextrose solution, Hank's solution, etc. Other additives may include additives to provide isotonicity, buffers, preservatives, and the like. The antibody or derivative thereof will usually be formulated in purified form at concentrations in the range of about 0.05 to 10 µg/ml. The antibody may be administered parenterally, typically intravenously or intramuscularly, as a bolus, intermittently or in a continuous regimen.

Desirably, the dose should deplete or at least bind about 75% of the undesired T-cells, preferably at least about 90%. Typical doses for adult humans will be in a range of about 10 to 100 mg. Doses for children may be extrapolated from the adult human dose based on relative body weight.

Instead of antibodies, oligopeptides may be employed, having the same or substantially the same sequence as the oligopeptide sequence identified as being diagnostic of multiple sclerosis. These sequences will be oligopeptides of at least 8, usually at least 10 and preferably at least 12 amino acids, and generally not more than about 50 amino acids of the T-cell receptor subunit chain. While the entire subunit(s) may be employed, usually, not more than about 50 number % of the amino acids will be employed, particularly excluding the conserved or constant region. All or at least a portion of the variable region, capable of binding to the target protein (the protein recognized by the T-cell receptor) and/or MHC antigen, will be present. The MHC antigen may be by itself or bound to a fragment of the target protein, which fragment will normally include the particular locus associated with the disease.

Of particular interest, is the demonstration that the variable rearrangements of T-cells associated with specific sites of multiple sclerosis, have a restricted repertoire, so that a relatively small number of T-cell variable regions of both the α and β subunits will be observed. Furthermore, as will be discussed below, a limited repertoire of MHC type will be associated with the disease and as to these repertoires, there will generally be a restricted repertoire of T-cell receptor variable regions.

With MS patients it is found in the brain, particularly in the plaques associated with the disease, that T-cells may be isolated which have rearranged germline DNA to provide for expression of the T-cell receptor. This may be contrasted with brains of normal healthy individuals, where the T-cells found in the brain have unrearranged germline DNA.

By identifying a particular Class II haplotype or molecular phenotype, one can then identify particular Vα and Vβ variable regions associated with multiple sclerosis . Once the autoimmune associated T-cell receptor or variable regions are identified, one may then use the various therapies which are described in the subject application for prophylaxis or treatment.

As previously indicated, of interest are specific V regions of the α chain of the T-cell receptor. For sequences of human and mouse V regions, see Concannon et al., Proc. Natl. Acad. Sci. USA (1986) 83:6598-6602. Of the regions of interest of CD4 T-cell associated with multiple sclerosis, among Vα families are 8-10, 12 and 16, particularly 10.

The oligopeptide may be joined to other peptides, proteins, or polyalkyleneoxy compounds for a variety of reasons, such as to provide for enhanced stability, toleration, ease of preparation or purification, or the like. The subject peptides may be used to inhibit the binding of the T-cell receptor to the target peptide.

The peptide may be formulated in substantially the same manner as described for the antibodies. The amount of the active ingredient administered will vary widely depending on the particular composition, the particular host, the number and frequency of administrations, the manner of administration, etc. Usually there will be from about 0.01 to 10 µg/kg of host, more usually from about 0.05 to 5 µg/kg of host, where the concentration may range from about 10 µg/ml to about 1 mg/ml.

The manner of administration may vary widely, depending upon the formulation and nature of the active ingredient. Administration may be parenteral, intravascular, peritoneally, subcutaneous, oral, etc., may employ catheters, pumps, constant diffusion membranes, etc.

The oligopeptides may be prepared in a variety of ways, conveniently, in accordance with conventional synthetic procedures. Where larger sequences are involved, such as 30 amino acids or more, recombinant DNA techniques may be employed, where the gene may be synthesized in accordance with conventional ways, such as commercially available DNA synthesizers, expanded employing the polymerase chain reaction, and then inserted into an appropriate vector having the necessary transcriptional and translational initiation and termination regions. The resulting vector is then transformed into a host in which the expression vector is replicated and functional expression is obtained. The product may be secreted and harvested from the medium or when not secreted and retained cytoplasmically, the cells are harvested, lysed, and the desired protein isolated and purified in accordance with conventional ways.

Instead of the oligopeptide, anti-idiotype antibodies may be employed. By preparing a monoclonal antibody to the idiotype of the antibody to the subject oligopeptide, the anti-idiotype may mimic the oligopeptide and serve to compete for the MHC with the T-cell receptor for the MHC antigen. The anti-idiotype may provide greater stability on administration, as well as other advantages.

The protective compositions may be used in vitro or vivo by adding to groups of cells comprising lymphocytes and cells associated with multiple sclerosis or target protein. By adding the protective composition, usually a protein such as an antibody or peptide having the appropriate variable region sequence, one can prevent the destruction of the cells and/or target protein. Where cells are involved, the T-cells will be restricted by the major histocompatibility antigen of the target cells, with the target cells usually being syngeneic with the T-cells.

In addition to the compositions of this invention, other compositions may be employed to enhance protection. These compositions may comprise oligopeptides, one or more different oligopeptides, comprising the following sequence: charged amino acid, two hydrophobic amino acids, and at least one of the next two amino acids being a polar amino acid, where the charged or polar amino acid may be substituted by glycine, usually not more than one being substituted by glycine. The charged amino acids are aspartic acid, glutamic acid, lysine, arginine, and histidine (D, E, K, R, H). The hydrophobic amino acids are alanine, proline, valine, leucine, isoleucine, methionine, phenylalanine, tryptophan, and tyrosine, that is both the aliphatic and aromatic neutral or substantially neutral amino acids having not more than one heteroatom, e.g., chalcogen, on the side chain (A, P, V, L, I, M, F, W, and Y). The polar amino acids will be the charged amino acids, as well as serine, threonine, asparagine, and glutamine (S, T, N, and Q).

The motif sequence may be part of a sequence of an immunogen of interest, associated with multiple sclerosis, there usually being more than one partial sequence in the immunogen comprising the subject motif. The oligopeptide comprising the subject motif may be from any site of the immunogen sequence, that is N-terminal or C-terminal proximal or central, where the oligopeptide sequence will normally be substantially homologous with from 9-15 amino acids of the immunogen sequence, although longer sequences may also be employed. Usually, the difference in homology between the natural sequence and the oligopeptide which is employed will be not more than two lesions, more usually not more than 1 lesion, which may be insertions, deletions, or conservative or non-conservative substitutions.

Usually, the motif sequence present in the oligopeptide will be at other than the C-terminus of the oligopeptide, desirably being at the N-terminus and not closer to the C-terminus than the center of the sequence, where the second, third, or fourth amino acid of the motif (depending upon whether there are four or five amino acids in the motif) is the central amino acid.

The compositions of this invention will include usually at least one sequence of an immunogen of interest including the subject motif and may include two or more oligopeptide motifs containing sequences of from about 9-15 amino acids present in the immunogen, depending upon the number of motifs present in the immunogen. Thus, if there area plurality of motifs present in the immunogen, all or fewer than all of the sequences including the motifs may be employed in a single composition. Usually, there will be not more than ten different motif comprising oligopeptides, more usually not more than about six different oligopeptides in the composition.

In preparing the subject compositions, one would select an immunogen related to multiple sclerosis against which an immune response of a host is to be modulated. The oligopeptide may serve to tolerize the host to prevent immune attack against the endogenous protein or cell producing the endogenous protein.

The particular protein of interest will be screened for the presence of the subject motif and one or more sequences including the motif selected. Where the histocompatibility genotype (haplotype) of the intended recipient is known, one sequence may be preferred over another. However, where the haplotype is not known, or the composition may be administered to a number of different hosts, it will frequently be desirable to combine a number of the sequences as oligopeptides in the same composition. The oligopeptides may be present as the individual peptides, or may be joined together in a single sequence, with or without intervening bridges, where any bridges will be other than the naturally occurring intervening sequences of the immunogen. Desirably, any such sequence would have fewer than about 100 amino acids, more usually fewer than about 60 amino acids.

The subject oligopeptides may be modified in a variety of ways. For toleration, the subject peptides may be conjugated to syngeneic spleen cells, or be linked to an innocuous immunogen to which the host has been previously immunized, such as tetanus toxoid, bovine serum albumin, etc. Adjuvants are normally avoided.

Sequences which may be employed for toleration will be sequences from proteins endogenous to the host involved with autoimmune diseases, which include such proteins as the neurological proteins found in the peripheral nervous system (PNS) or the central nervous system (CNS) and the acetylcholine receptor (AChR). These proteins are designated as Pₒ which is found in the PNS and CNS, P1, in myelin basic protein, the predominant CNS protein of myelin, P2, a predominant PNS myelin protein, PLP, a proteolipid protein, a PNS and CNS myelin constituent, and the acetylcholine receptor. P1 is involved in post-immunization encephalomyelitis and may be involved in multiple sclerosis. P2 is involved in post-immunization neuritis (Guillain-Barre syndrome) a major complication, for example, in the swine flu immunization program and the acetylcholine receptor is involved in myasthenia gravis and may play a role in post-immunization myositis. These protein fragments may be modified by one or more lesions to maintain the binding affinity for the MHC antigen while substantially reducing the binding affinity to the T-cell receptor.

Transplantation or MHC antigens have polymorphic regions, where the individual alleles are associated with specific hosts. For the most part, the host will be diploid and heterozygous, so that each host will have two haplotypes, meaning that there will be two different copies of a particular transplantation antigen type from the same locus, unless the host is homozygous at that particular locus. Therefore, as to an individual host or a plurality of hosts, mixtures of oligopeptides will usually be employed. The subject oligopeptides may be administered concurrently or consecutively with the oligopeptides of the T-cell receptor. The subject oligopeptides may be administered in a variety of ways, by themselves or in conjunction with various additives. Various carriers may be employed which are physiologically acceptable, such as water, alcohol, saline, phosphate buffered saline, sugar, mineral oil, etc. Other additives may also be included, as stabilizers, detergents, flavoring agents, thickeners, etc. The amount of active ingredient administered will vary widely depending upon the particular composition, the particular host, the number and frequency of administrations, the manner of administration, etc. Usually, there will be from about 0.01-10 µg/kg of host more usually from about 0.05-5 µg/kg of host, where the concentration may range from 10 µg/ml-1 mg/ml.

In addition to identifying T-cell variable regions associated with multiple sclerosis, one may in addition identify specific molecular phenotypes associated with susceptibility to said autoimmune disease. The phrase "molecular phenotype" is used instead of the designation "haplotype" since in the absence of segregation analysis in families, it cannot be certain whether these genes are all in a cis configuration on a single chromosome. A phenotype associated with multiple sclerosis is DRB1 1501, DQA1 0102 and DQB1 0602. This phenotype may be further broken down into 1a, associated with DPB1 0401 and 1b 0402. This particular phenotype is associated with Vβ family rearrangements, particulary Vβ 5.1, 5.2 and 6.

By identifying MHC molecular phenotypes of individuals suffering multiple sclerosis, one can establish certain molecular phenotypes which provide for a susceptibility to the disease. In the case of multiple sclerosis one can look to see for rearrangement of T-cells and the prevalence of the various families and members of the families of the Vβ and Vα T-cell receptors. Once these are identified, one can use this information to ablate the T-cells associated with the disease. Thus, by isolating diseased tissue, e.g., plaques, and identifying T-cells having rearrangements, one can identify a family of Vβ and Vα regions which are associated with the disease, so that once the molecular phenotype has been identified as providing susceptibility to autoimmune disease one will also know which T-cells to oblate or target.

References to the V_{α} families 1,2 and 7 and to the V_{β} families 5.1, 5.2 or 6 are for comparative purposes.

The following examples are offered by way of illustration and not by way of limitation.

### EXPERIMENTAL

### TcR Vα expression in brain plaques of multiple sclerosis

Samples were taken from brain plaques of 3 patients with chronic progressive MS, and 3 controls (non MS). Total RNA and cDNA (from 5 µg RNA) were prepared according to standard procedures. cDNA was also prepared from 1 µg RNA isolated from a pool of peripheral blood lymphocytes from five different individuals, stimulated with 3 µg/ml of PHA. cDNAs were amplified by PCR for 40 cycles in the presence of 10 µCi of [³²P]dATP (Amersham). Samples were analyzed by gel electrophoresis with ethidium bromide to identify the specific fragment band. After separation, bands were excised and radioactivity was determined. Results are expressed in median cpm. All TcR 5' primers amplify TCR sequences from germ line DNA using a specific 3' Vα primer for each family. The following tables indicate the primers employed and the results.

**TABLE 1**

| T-CELL RECEPTOR α PRIMERS | | | |
|---|---|---|---|
| Primer | Clone | Sequence | Family members |
| Vα 1 | HAP 10 | 5'-TTGCCCTGAGAGATGCCAGAG-3' | 1.1, 1.2, 1.3 |
| Vα 2 | HAP 26 | 5'-GTGTTCCCAGAGGGAGCCATTGCC-3' | 2.1, 2.2 |
| Vα 3 | HAP 05 | 5'-GGTGAACAGTCAACAGGGAGA-3' | 3.1 |
| Vα 4 | HAP 08 | 5'-ACAAGCATTACTGTACTCCTA-3' | 4.1 |
| Vα 5 | HAP 35 | 5'-GGCCCTGAACATTCAGGA-3, | 5.1 |
| Vα 6 | HAP 01 | 5'-GTCACTTTCTAGCCTGCTGA-3' | 6.1 |
| Vα 7 | HAP 21 | 5'-AGGAGCCATTGTCCAGATAAA-3' | 7.1, 1.2 |
| Vα 8 | HAP 41 | 5'-GGAGAGAATGTGGAGCAGCATC-3' | 8.1, 1.2 |
| Vα 9 | HAP 36 | 5'-ATCTCAGTGCTTGTGATAATA-3' | 9.1 |
| Vα 10 | HAP 58 | 5'-ACCCAGCTGGTGGAGCAGAGCCCT-3' | 10.1 |
| Vα 11 | HAP 02 | 5'-AGAAAGCAAGGACCAAGTGTT-3' | 11.1 |
| Vα 12 (Ab13) | PGA 5 | 5'-CAGAAGGTAACTCAAGCGCAGACT-3' | 12.1 |
| Vα 13 | AB 11 | 5'-GCTTATGAGAACACTGCGT-3' | 13.1 |
| Vα 14 | AB 21 | 5'-GCAGCTTCCCTTCCAGCAAT-3' | 14.1 |
| Vα 15 | AC 24 | 5'-AGAACCTGACTGCCCAGGAA-3' | 15.1 |
| Vα 16 | AE 212 | 5'-CATCTCCATGGACTCATATGA-3' | 16.1 |
| Vα 17 | AF 211 | 5'-GACTATACTAACAGCATGT-3' | 17.1 |
| Vα 18 | AC 9 | 5'-TGTCAGGCAATGACAAGG-3' | 18.1 |
| Cα (Ab51) | PGA 5 | 5'-AATAGGTCGACACACTTGTCACTGGA-3' | Cα |

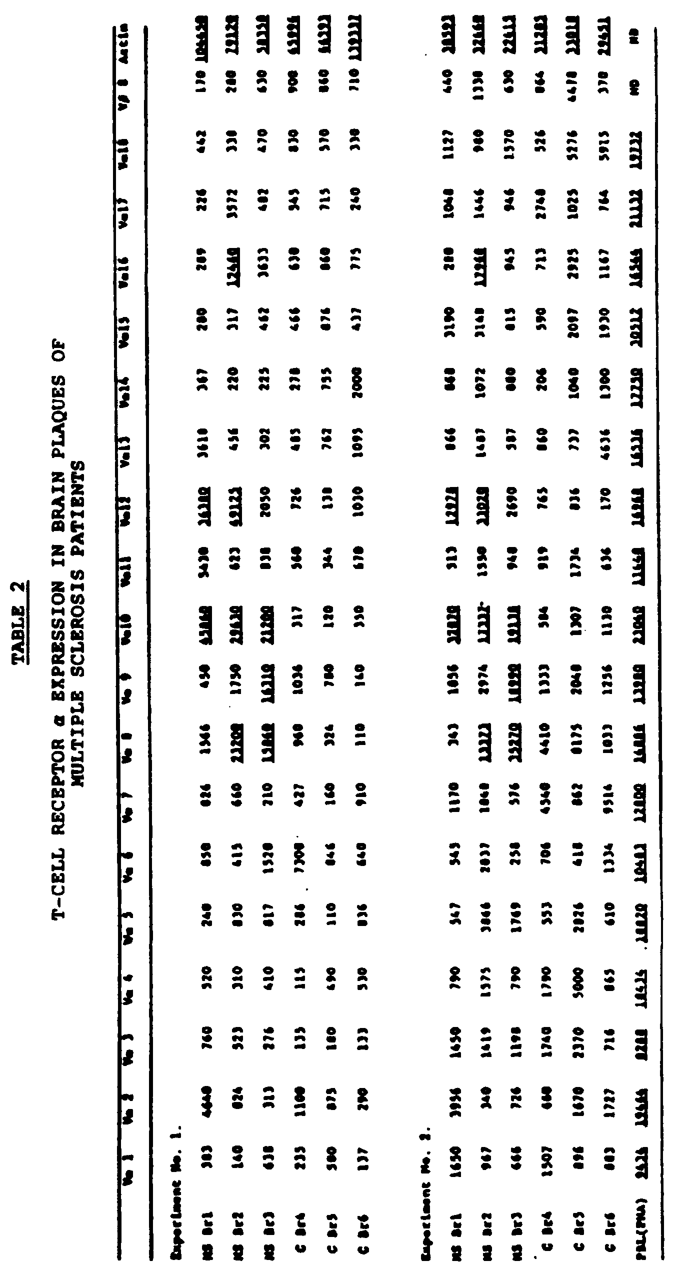

The T-cell receptors present in the brain of MS patient 1 were amplified and subjected to gel electrophoresis, where control brain cDNA, MS parietal region brain cDNA, MS occipital region brain cDNA, PGA5, a full length TcR α cDNA (Sim et al., Nature 312, (1984) 771-775) were compared. Two µl of cDNA was combined in a 100 µl reaction volume, with 1 unit of DNA Taq polymerase (Perkin Elmer-Cetus), 10 µl 10x reaction buffer, 50 µM each dNTPs, and 1µm of each primer. The PCR profile used was: denaturation 96°C for 60 sec., annealing 45°C for 60 sec. and extension 72°C for 120 sec., for a total of 35 cycles on a DNA Thermal Cycler (Perkin Elmer-Cetus). One tenth of each sample was independently run in a 4% Nusiev gel, and an appropriate size fraction was cut from the gel. The agarose piece was frozen and thawed 3 times, and 2 µl of the supernatant were directly reamplified with the same primers for an additional 25 cycles. Actin sequences were successfully amplified from brain cDNA, but not from the PGA 5 control using the following primers: (5'-ACGAAGACGGACCACCGCCCTG-3', 5'-CACGTTGTGGGTGACGCCGTC-3'). Vα and Cα transcripts were amplified from both MS brain cDNA and PGA 5 templates, but not from the control MS brain cDNA with primers AB 13-14 (5'-CAGAAGGTAACTGCAGCGCAGACT-3', 5'TTGGGGATCCAGAGCACAGAAGTATACTGC-3'), which include the restriction sites PstI and BamHI and define a 286 bp fragment of the Vα12.1 region gene and AB 41-42 (5'CAGAACCCTGACCCTGCCGTGTAC-3',5'-GTGTCCACAGTTTAG-GTTCGTATCTGT-3', which include a SalI site and define a bp fragment of the Cα region transcript) respectively. Note that rearranged TcRα sequences could be amplified from cDNA of the MS brain prepared from the occipital region using the Vα12.1 primer AB 13 and Cα primer AB 42.

Junctional region sequences were derived from the Vα12.1-JC amplification from the occipital region of the MS brain. 100 µl of the PCR reaction were phenol:chloroform extracted twice with 1/1 volume, chloroform extracted once with 1/1 volume, and dialyzed through a Centricon 30 (Amicon) with 2 ml of TE buffer for 30 min at 5000 rpm. The sample was recovered and the DNA digested for 3 h with BamHI and PstI (or SalI). After digestion, the sample was phenol:chloroform extracted and then chloroform extracted once, passed through the same Centricon column as described above, and brought to a final volume of 20 µl. 3 µl of sample was put into a 10 µl ligation with 200 ng of Bam HI/PstI or BamHI/SalI cut M13mp18 and ligated overnight at 16°C with T4 DNA ligase (New England Biolabs). Transformation into E. coli JM101 was done according to standard procedures and positive plaques were selected by hybridization to ³²P-labelled TcR probes. 30 clones were sequenced by the dideoxy chain termination method using ³⁵S-dATP and Sequenase® (U.S. Biochemicals).

The above results showed in the amplification of the cDNA of one patient and the actin control, that actin could be amplified from the brain cDNAs but not from PGA5, a full length cDNA clone which contains the Vα12.1 segment. Also, evident were lesser amounts of a smaller PCR product corresponding to the Vα12.1 gene in the patient but not in the control sample. To ensure that the one Vα family was amplified, genomic and brain Vα12.1 PCR products were analyzed using restriction endonucleases and compared to the known restriction map. Only the expected fragments were observed, consistent with the notion that only the Vα12.1 family was amplified. When colonies containing cloned Vα PCR products were screened with a Vα12.1 region probe, approximately 20% were positive. DNA from several of these colonies was sequenced and found to be identical to the published Vα12.1 sequences (Sim et al., Nature (1984) 312, 771-775). Thus, the restriction fragment length polymorphisms (RFLP) recently associated with MS susceptibility must be in a sequence flanking to the TcR Vα gene. (Oksenberg et al., Proc. Natl. Acad. Sci. USA 86 988-992 (1989)

The above results demonstrated that PCR could amplify the receptor transcripts from post-mortem brain samples, starting from 5 µg of total RNA without the necessity of in vitro expansion of T-cells. Similarly, Cα sequences were amplified from MS brain cDNAs, but not from the control sample. A subsequent amplification using primers complementary to the Vα and the Cα TcR regions produced a major band when the control PGA5 and cDNA from the occipital region of the MS brain were used as a template, indicating the presence of rearranged TcR transcripts in the sample. The Vα and Cα amplifications from the parietal region brain library most probably represent real transcripts from rearranged chromosomes, as has been found in other cDNA libraries from T-cell lines (Loh et al., Science (1989) 243, 217-220.

No PCR product was observed using primers corresponding to the Vβ8 family, even though these primers are able to amplify the gene from buffy coat extracted genomic DNA. This TcR V region was recently reported to be associated with susceptibility to MS (Beall et al., J. Neuroimmunol (1989) 21, 59-66.

To provide further evidence that the DNA produced during the PCR amplification was an authentic amplified product of rearranged TcR genes, the PCR products were sequenced after double screening of colonies with Vα and Cα probes. Only two different J regions were seen in the thirty sequences examined, both different from the PGA5 Jα sequence. Eleven sequences contain the Jα O family found in clone HAP41 (Yoshikai et al. J. Exp. Med. (1986) 164, 90-103. Fourteen sequences had a previously undescribed Jα sequence. (Described in the text.)

In order to completely analyze the TcR Vα usage in MS brains, 18 different Vα specific oligonucleotides for use as 5' PCR primers, based on published sequences for these gene families were prepared (Yoshikai et al. 1986, supra; Kline et al., Proc. Natl. Acad. Sci. USA (1987) 84, 6884-6888. Optimal conditions for amplification with each primer were ascertained with genomic DNA using a specific 3' Vα primer for each TcR Vα family and with reverse-transcribed RNA isolated from PHA stimulated peripheral blood lymphocytes. Using 5' Vα primers and a common 3' Cα primer, the results from amplification of brain cDNA show that in each brain only a few TcR V gene families are preferentially expressed and rearranged. The Vα 10 and 12 were detected in MS brains 1 and 2. MS brain 2 also expressed the Vα 8 and the Vα 16. In MS brain 3, the Vα 8, 9 and 10 families were efficiently amplified. The Vα 10 was thus common to all three samples.

TCR Vα and Vβ rearrangements were studied in 16 MS brains and in 10 control brains. TCRVα-Jα-Cα and Vβ-Dβ-Jβ-Cβ rearrangements were confirmed with Southern blotting and hybridization of the PCR product obtained by amplification with 1 of 18 Vα or 1 of 21 Vβ specific oligonucleotide primers. The following table indicates the primers.

**TABLE 4**

| T-CELL RECEPTOR α PRIMERS | | | |
|---|---|---|---|
| PRIMER | SEQUENCE | PRIMER | SEQUENCE |
| Vα 1 | 5'-TTGCCCTGAGAGATGCCAGAG-3' | Vβ 1 | 5'-GCACAACAGTTCCCTGACTTGCAC-3' |
| Vα 2 | 5'-GTGTTCCAGAGGGAGCCATTGCC-3' | Vβ 2 | 5'-TCATCAACCATGCAAGCCTGACCT-3' |
| Vα 3 | 5'-GGTGAACAGTCAACAGGGAGA-3' | Vβ 3 | 5'-GTCTCTAGAGAGAAGAAGGAGCGC-3' |
| Vα 4 | 5'-ACAAGCATTACTGTACTCCTA-3' | Vβ 4 | 5'-ACGATCCAGTGTCAAGTCGT-3' |
| Vα 5 | 5'-GGCCCTGAACATTCAGGA-3' | Vβ 5.1 | 5'-ATACTTCAGTGAGACACAGAGA-3' |
| Vα 6 | 5'-GTCACTTTCTAGCCTGCTGA-3' | Vβ 5.2 | 5'-TTCCCTAACTATAGCTCTGGCTG-3' |
| Vα 7 | 5'-AGGAGCCATTGTCCAGATAAA-3' | Vβ 6 | 5'-AGGCCTGAGGGATCCGTCTC-3' |
| Vα 8 | 5'-GGAGAGAATGTGGAGCAGCATC-3' | Vβ 7 | 5'-CCTGAATGCCCCAACAGCTCTC-3' |
| Vα 9 | 5'-ATCTCAGTGCTTGTGATAATA-3' | Vβ 8 | 5'-ACTTTAACAACAACGTTCCGA-3' |
| Vα 10 | 5'-ACCCAGCTGCTGGAGCAGAGCCCT-3' | Vβ 9 | 5'-CTAAATCTCCAGACAAAGCTCAC-3' |
| Vα 11 | 5'-AGAAAGCAAGGACCAAGTGTT-3' | Vβ 10 | 5'-TCCAAAAACTCATCCTGTACCT-3' |
| Vα 12 | 5'-CAGAAGGTAACTCAAGCGCAGACT-3' | Vβ 11 | 5'-TGTTCTCAAACCATGGGCCATGAC-3' |
| Vα 13 | 5'-GCTTATGAGAACACTGCGT-3' | Vβ 12 | 5'-GATACTGACAAAGGAGAAGTCTCAGAT-3' |
| Vα 14 | 5'-GCAGCTTCCCTTCCAGCAAT-3' | Vβ 13 | 5'-GGTGAGGGTACAACTGCC-3' |
| Vα 15 | 5'-AGAACCTGACTGCCCAGGAA-3' | Vβ 14 | 5'-ACCCAAGATACCTCATCACAG-3' |
| Vα 16 | 5'-CATCTCCATGGACTCATATGA-3' | Vβ 15 | 5'-AGTGTCTCTCGACASGGCACAG-3' |
| Vα 17 | 5'-GACTATACTAACAGCATGT-3' | Vβ 16 | 5'-CATGATAATCTTTATCGACGTGTT-3' |
| Vα 18 | 5'-TGTCAGGCAATGACAAGG-3' | Vβ 17 | 5'-AGCCCAATGAAAGGAACACAGTCAT-3' |
| | | Vβ 18 | 5'-AGCCCAATGAAAGGACACAGTCAT-3' |
| | | Vβ 19 | 5'-ACCCCCGAAAAAGGACATACT-3' |
| | | Vβ 20 | 5'-CTCTGAGGTGCCCCAGAA-3' |
| Cα | 5'-AATAGGTCGACAGACTTGTCACTGGA-3' | Cβ | 5'-TTCTGATGGCTCAAACAG-3' |

Coded human brain samples were obtained from the Rocky Mountain Multiple Sclerosis Center in Englewood, Colorado and La Trobe University, Victoria, Australia. Samples included rapidly frozen and cryopreserved autopsy samples from different regions of 16 MS brains and 10 non-MS controls. Each sample was homogenized and the total RNA was extracted using the RNAzol method (Cinna/Biotecx, Friendswood, TX), (Chonzynski and Sacchi, Anal. Biochem. (1987) 162:156). Approximately 0.25 µg of total RNA was reverse transcribed into a first cDNA strand in a 10 µl reaction containing 1 µl 10xPCR buffer (100 mM Tris-HCl pH 8.3, 500 mM KCl, 15mM MgCl₂, 0.01% (w/v) gelatin (Perkin, Elmer, Norwalk, CT), 1 µl of 10 mM dioxynucleotide triphosphates, 0.25U random hexamers (Pharmacia, Piscataway, NJ) and 100U of Superscript MuLV-reverse transcriptase (BRL, Gaithersburg, MD). The reaction mix was incubated at room temperature for 10 min, followed by incubations at 42°C for 45 minutes and 95°C for 5 minutes. The mix was then quick chilled on ice. cDNA was subjected to enzymatic amplification by the PCR method. 10µl cDNA was combined in a 50 µl reaction mix with 4 µl 10x PCR buffer, 1.25U Taq polymerase, 0.5 µM of Cα or Cβ primer and 0.5 µM of Vα or Vβ specific oligonucleotide primer (Table 4).

The PCR profile used was: Denaturation 95°C for 60 s annealing 55°C for 60 sec and extension 72°C for 60 s for 35 cycles in a DNA Thermal Cycler.

DNA Isolation and HLA Typing: High molecular weight DNA was extracted from tissue samples according to standard procedures. HLA-DRB1, DQA1, DQB1 and DPB1 typing was performed by PCR, dot blotting and hybridization with allele specific oligonucleotide probes (Helmuth et al., Am. J. Hum. Genet. (1990) 47:515; and Bugawan et al., Immunogenetics (1990) 32:231).

Specificity was confirmed by identifying single rearrangements in antigen specific T-cell clones for B. burgdorferi, pertussis toxin and acetylcholine receptor. No amplification was detected in a colon carcinoma cell line. All samples were coded with the molecular biologist blinded to the origin of the specimen other than 3 MS brains and 3 controls.

A limited number of TCR Vα gene arrangments were seen in 15 of 16 of the MS specimens.

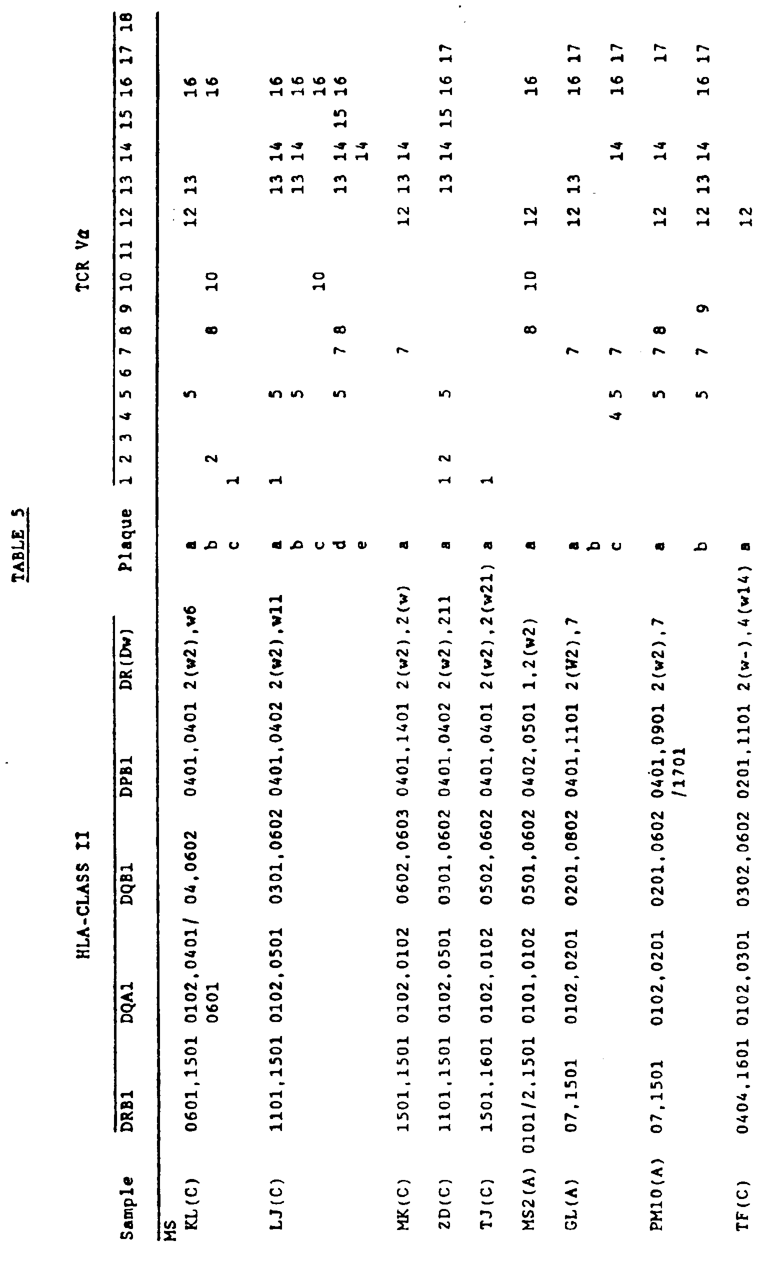

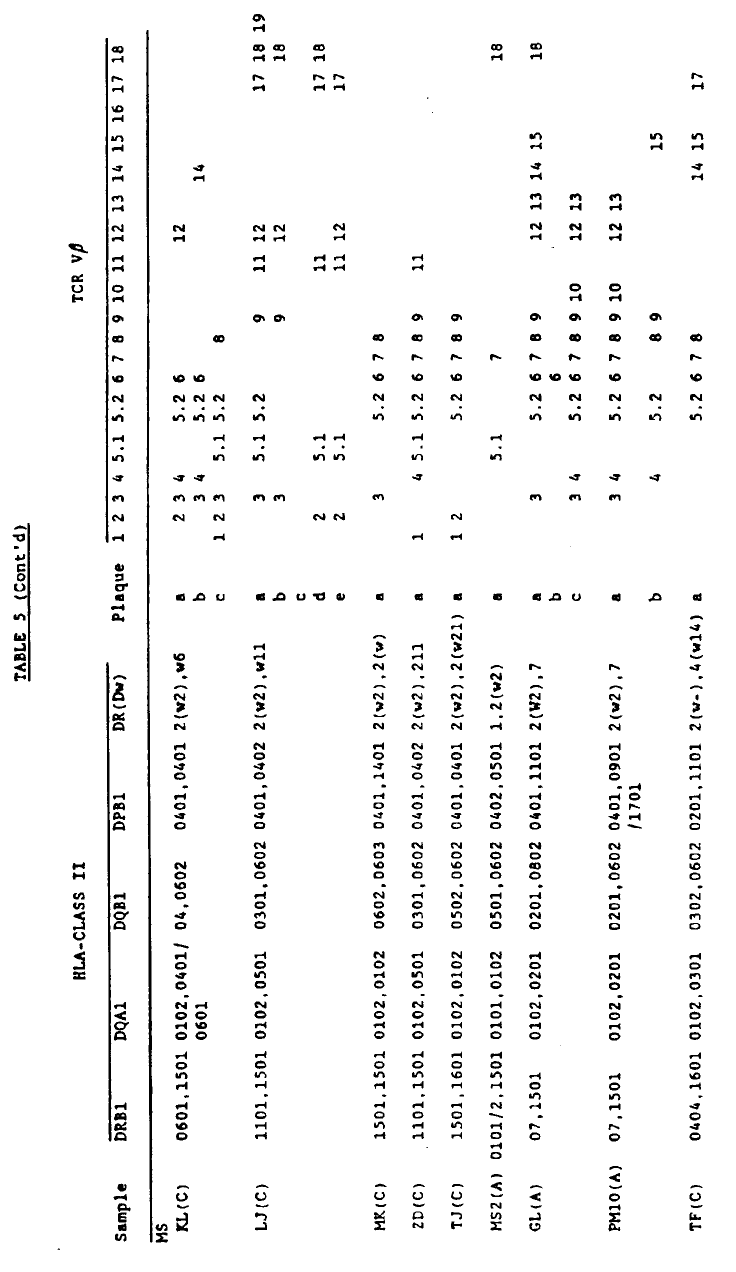

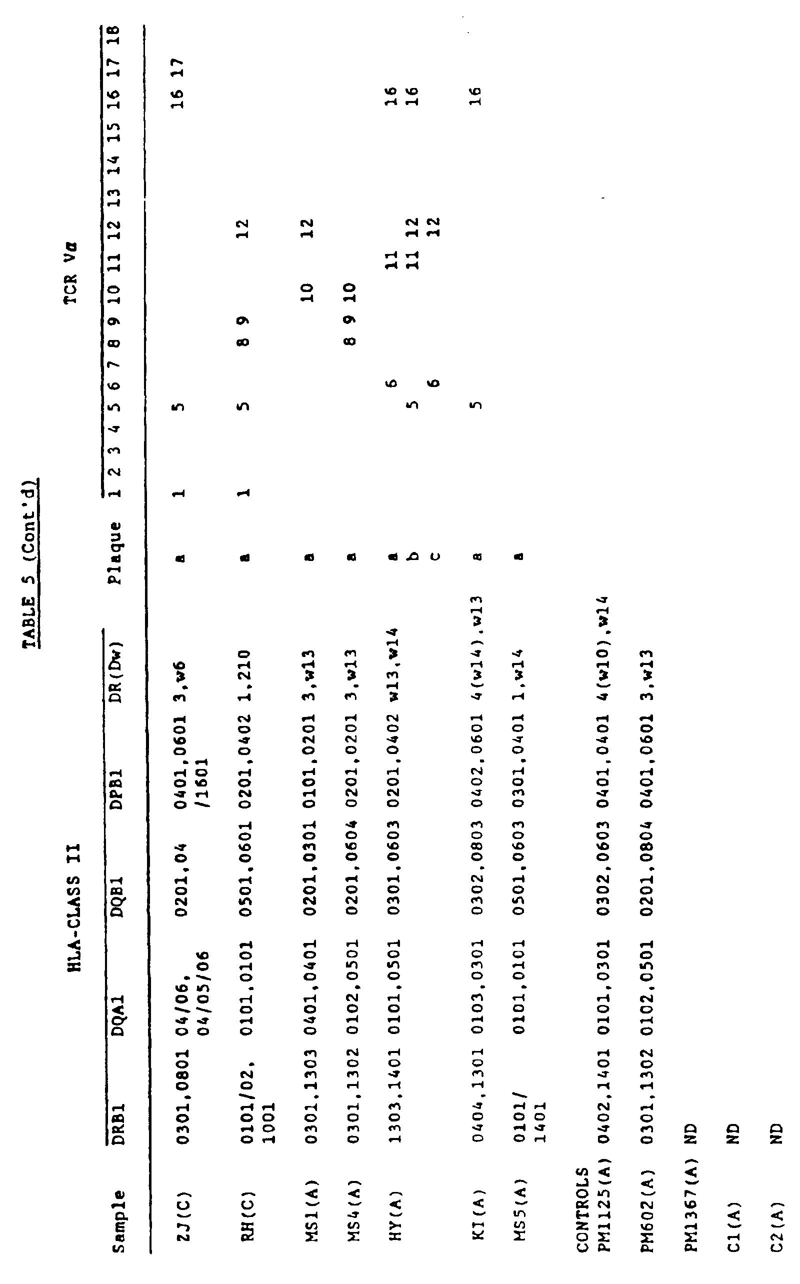

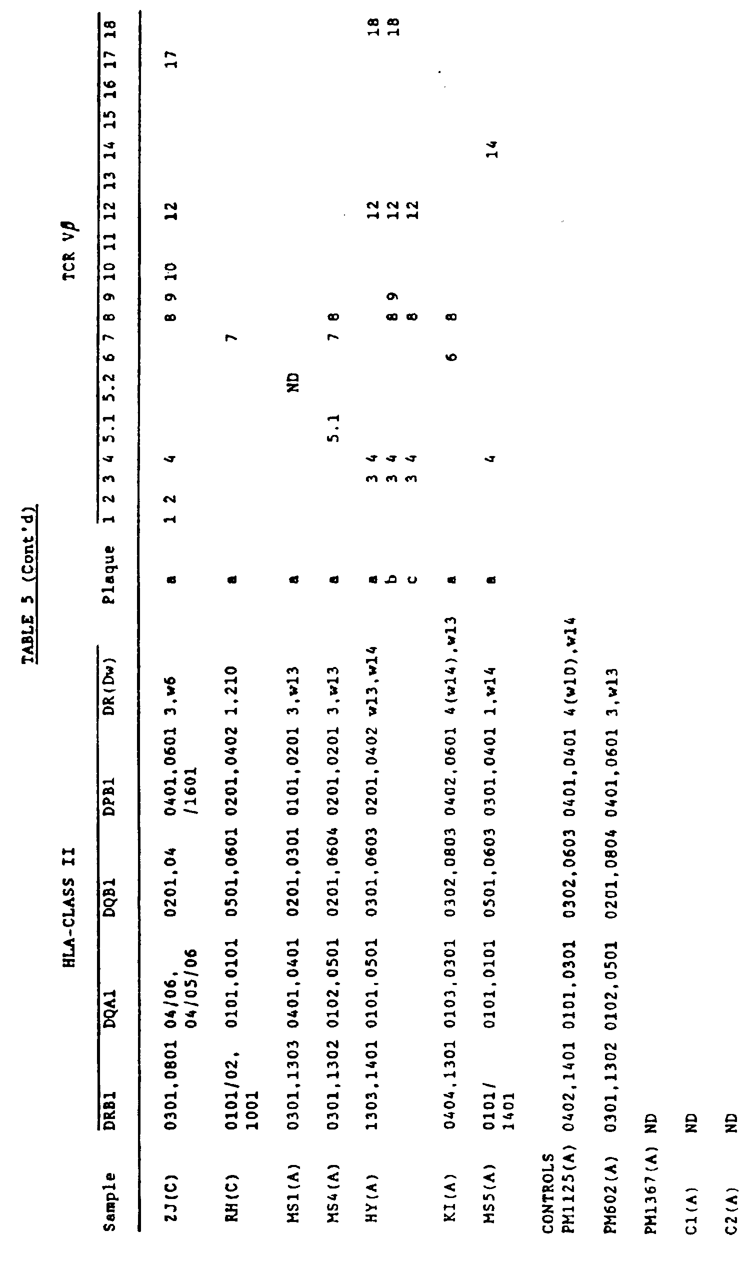

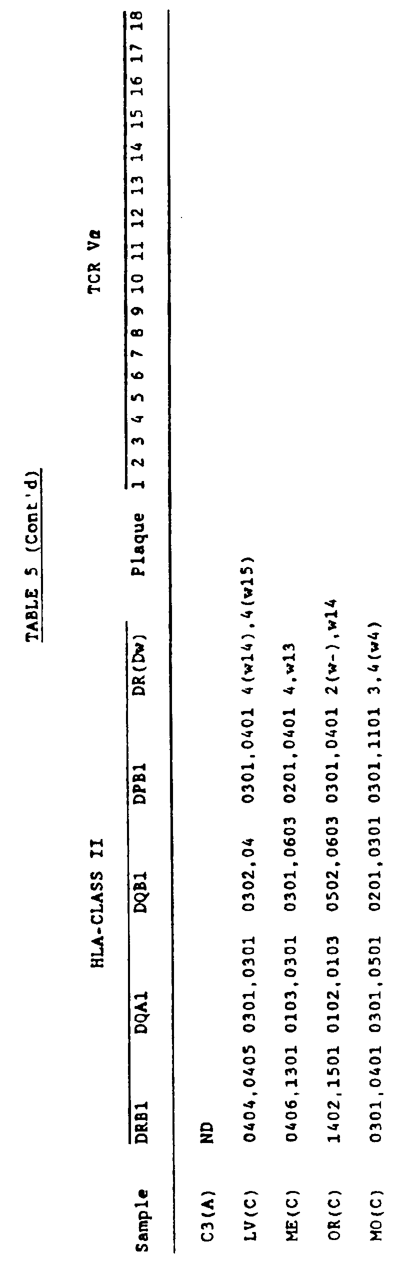

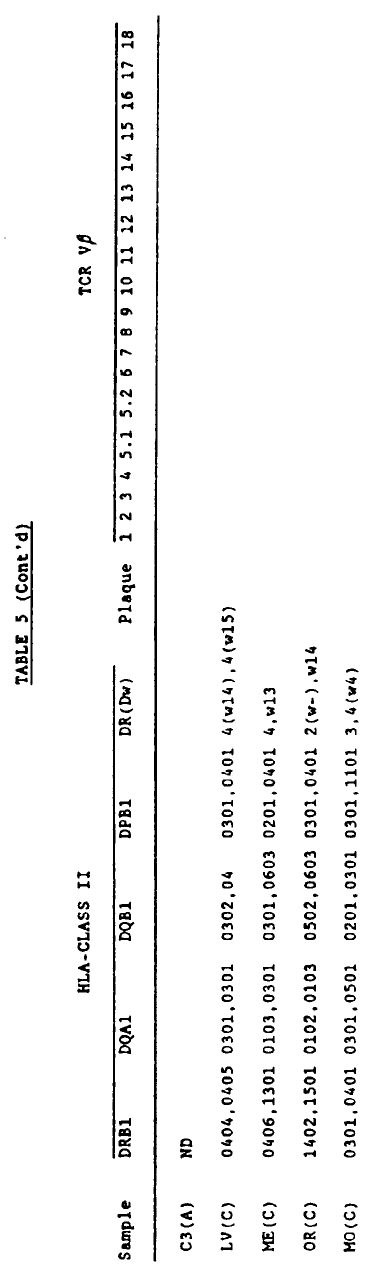

In one specimen, no Vα genes of the 18 families surveyed were rearranged, although this patient had two Vβ rearrangements. The number of TCR Vα genes transcribed ranged from 0 to 9 per brain, with a mean of 4.4 ± 2.8 (± 1 SD). TCR Vβ rearrangments were more diverse, with a range of 2 to 13 per brain, with a mean of 7.0 ± 3.4 (± 1 SD). TCR Vα or Vβ transcripts were not detected in any of the 10 brains of individuals who died of non-neurologic diseases.

All of the 16 MS patients were typed for the Class II HLA loci HLA-DRB1, DQA1, DQB1 and DPB1 using PCR and sequence-specific oligonucleotide probe hybridization. Eight of 16 patients were DRB1*1501, DQA1*0102, DQB1*0602 and either DPB1*0401 or 0402. This molecular HLA-DR:DQ haplotype, which corresponds to the cellular type HLA-DR2Dw2, is associated with increased susceptibility to MS in certain caucasoid populations. Patients who were of the above indicated molecular phenotype showed an increased frequency of certain Vα and Vβ rearrangements.

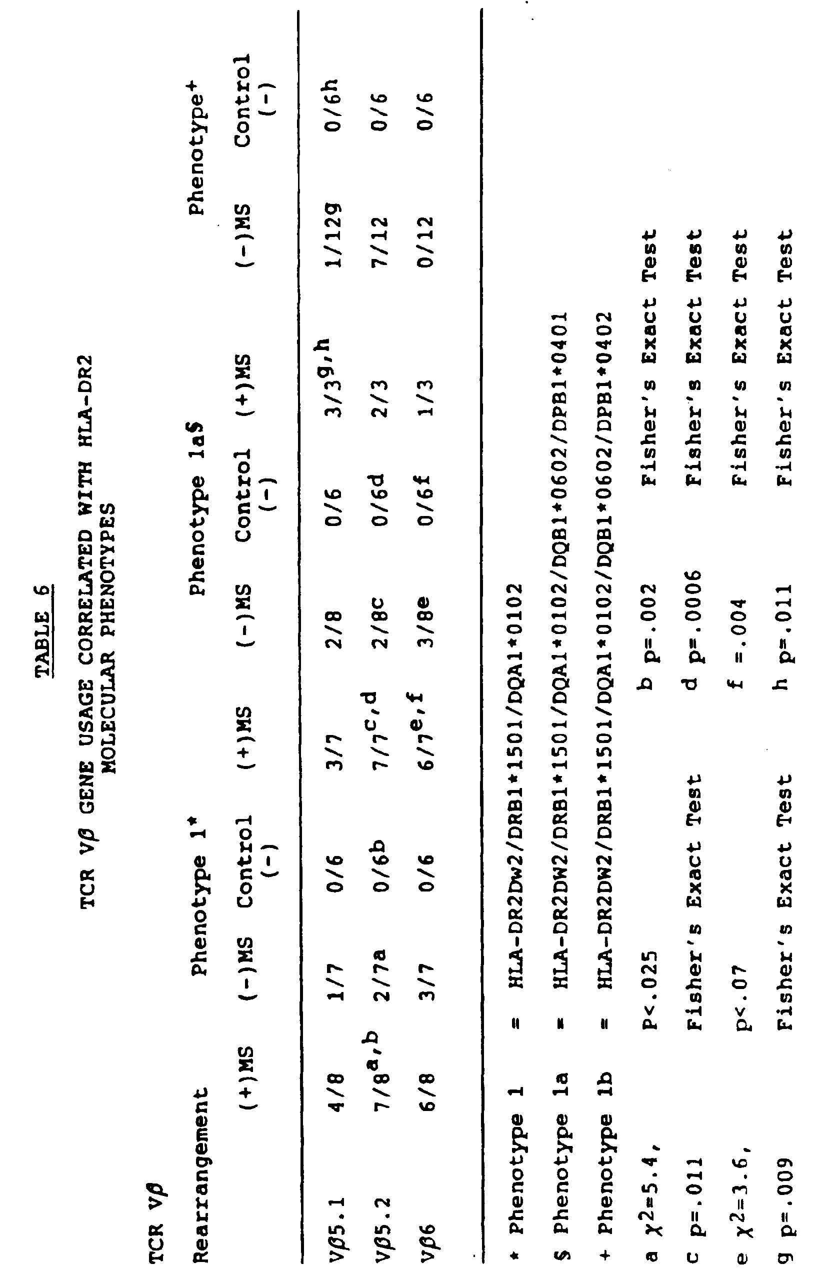

As evidenced by the above table, of 8 patients with the indicated phenotype, 7 had rearrangments of Vβ 5.2 and all 8 rearranged either Vβ 5.1 or 5.2 or both. Vβ 6 was transcribed in 6 of 8 MS brains with the above phenotype compared to 3 of 7 MS brains of patients who were not of the indicated phenotype and 6 control brains which were not of the indicated phenotype. Other frequently rearrangements seen in patients with the indicated phenotype were Vβ 7, 6/8, Vβ 12, 4/8, Vα 16, 6/8, Vα 5, Vα 7, Vα 12 and Vα 1 in 4/8.

It is evident from the above results that the composition of the present invention can be used in a method for identifying T-cell receptors associated with degenerative disorders. Thus, by a simple screening technique, one may identify those T-cell receptors which cause or combat disease and by various procedures inhibit or enhance their activity. The subject invention provides the capability to diagnose individuals susceptible to degenerative diseases associated with T-cell receptor variable regions. By screening degenerative tissue for T-cell receptors and identifying the specific T-cell receptors associated with that tissue and the HLA of the particular host, the relationship between the T-cell receptor, HLA and the disease may be established. Contrastingly, when the T-cells are associated with combatting a neoproliferative disorder, the particular T-cells may be employed for prophylaxis or therapy.

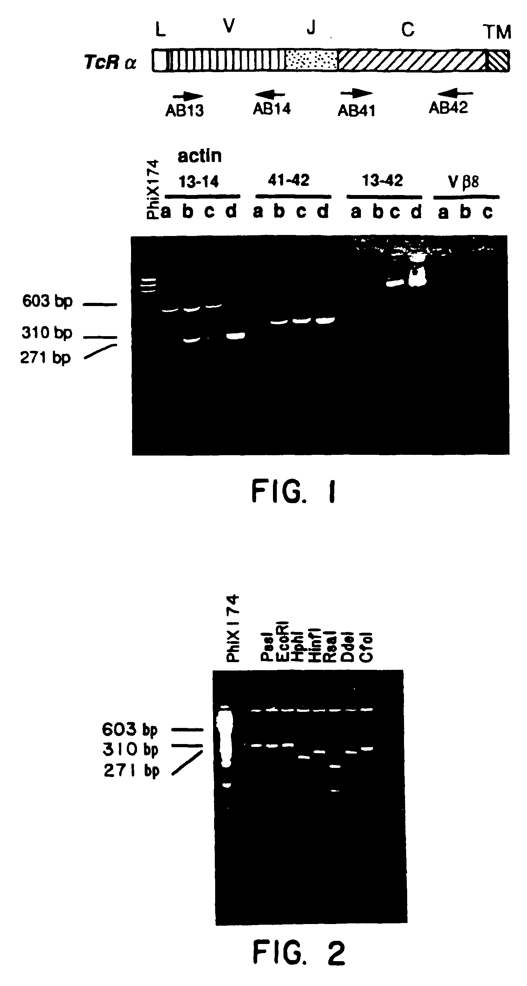

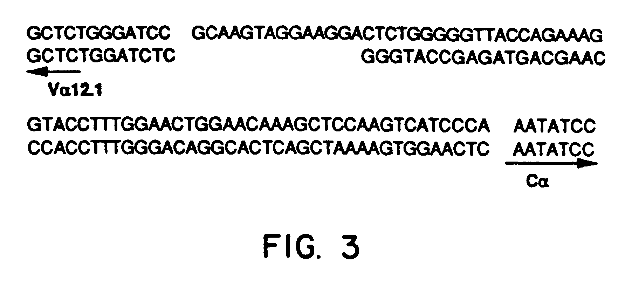

## Claims (Claims for the following Contracting State(s): AT, BE, CH, LI, DE, DK, FR, GB, IT, LU, NL, SE)

1. A composition for use in a method for treating a human being suffering from multiple sclerosis as a result of attack by T-cells of native tissue, said composition comprising an antibody specific for a variable region of the α subunit of a human T-cell receptor, said region belonging to one of the V_{α} families 8-10, 12 or 16, or combinations thereof and being capable of inhibiting the activity of T-cells having said T-cell receptor or subunit thereof.

2. A composition according to claim 1, wherein said antibody is cytotoxic.

3. A composition according to claim 2, wherein said family is the V_{α} 10 family.

4. A composition for use in a method for treating a human being suffering from multiple sclerosis as a result of attack by T-cells of native tissue, said composition comprising an anti-idiotype antibody specific for an antibody specific for a variable region of the α subunit of a human T-cell receptor, said region belonging to one of the V_{α} families 8-10, 12 or 16, or combinations thereof and being capable of inhibiting the activity of T-cells having said T-cell receptor or subunit thereof.

5. A composition for use in a method for treating a human being suffering from multiple sclerosis as a result of attack by T-cells of native tissue, said composition comprising:
an oligopeptide having substantially the same sequence as a variable region of the α subunit of a T-cell receptor, said region belonging to one of the human V_{α} families 8-10, 12 or 16 ;
and being capable of inhibiting the activity of T-cells having said T-cell receptor or subunit thereof.

6. A composition according to claim 5, wherein said family is the V_{α} 10 family.

## Claims (Claims for the following Contracting State(s): ES)

1. A composition for use in a method for treating a human being suffering from multiple sclerosis as a result of attack by T-cells of native tissue, said composition comprising an antibody specific for a variable region of the α subunit of a human T-cell receptor, said region belonging to one of the V_{α} families 8-10, 12 or 16, or combinations thereof and being capable of inhibiting the activity of T-cells having said T-cell receptor or subunit thereof.

2. A composition according to claim 1, wherein said antibody is cytotoxic.

3. A composition according to claim 2, wherein said family is the V_{α} 10 family.

4. A composition for use in a method for treating a human being suffering from multiple sclerosis as a result of attack by T-cells of native tissue, said composition comprising an anti-idiotype antibody specific for an antibody specific for a variable region of the α subunit of a human T-cell receptor, said region belonging to one of the V_{α} families 8-10, 12 or 16, or combinations thereof and being capable of inhibiting the activity of T-cells having said T-cell receptor or subunit thereof.

5. A composition for use in a method for treating a human being suffering from multiple sclerosis as a result of attack by T-cells of native tissue, said composition comprising:
an oligopeptide having substantially the same sequence as a variable region of the α subunit of a T-cell receptor, said region belonging to one of the human V_{α} families 8-10, 12 or 16 ;
and being capable of inhibiting the activity of T-cells having said T-cell receptor or subunit thereof.

6. A composition according to claim 6, wherein said family is the V_{α} 10 family.

7. A process for preparing a composition for use in a method for treating a human being suffering from multiple sclerosis as a result of attack by T-cells of native tissue, comprising mixing an antibody specific for a variable region of the α subunit of a human T-cell receptor, said region belonging to one of the V_{α} families 8-10, 12 or 16, or combinations thereof and being capable of inhibiting the activity of T-cells having said T-cell receptor or subunit thereof, and a pharmaceutically acceptable carrier and/or diluent.

8. A process according to claim 7, wherein said antibody is cytotoxic.

9. A process according to claim 8, wherein said family is the V_{α} 10 family.

10. A process for preparing a composition for use in a method for treating a human being suffering from multiple sclerosis as a result of attack by T-cells of native tissue comprising mixing an anti-idiotype specific antibody for a variable region of the α subunit of a human T-cell receptor, said region belonging to one of the V_{α} families 8-10, 12 or 16, or combinations thereof and being capable of inhibiting the activity of T-cells having said T-cell receptor or subunit thereof, and a pharmaceutically acceptable carrier and/or diluent.

11. A process for preparing a composition for use in a method for treating a human being suffering from multiple sclerosis as a result of attack by T-cells of native tissue comprising mixing an oligopeptide having substantially the same sequence as a variable region of the α subunit of a T-cell receptor, said region belonging to one of the human V_{α} families 8-10, 12 or 16, and being capable of inhibiting the activity of T-cells having said T-cell receptor or subunit thereof and a pharmaceutically acceptable carrier and/or diluent.

12. A process according to claim 11, wherein said family is the V_{α} 10 family.

## Claims (Claims for the following Contracting State(s): GR)

1. A process for preparing a composition for use in a method for treating a human being suffering from multiple sclerosis as a result of attack by T-cells of native tissue, comprising mixing an antibody specific for a variable region of the α subunit of a human T-cell receptor, said region belonging to one of the V_{α} families 8-10, 12 or 16 or combinations thereof and being capable of inhibiting the activity of T-cells having said T-cell receptor or subunit thereof, and a pharmaceutically acceptable carrier and/or diluent.

2. A process according to claim 1, wherein said antibody a cytotoxic.

3. A process according to claim 2, wherein said family is the V_{α} 10 family.

4. A process for preparing a composition for use in a method for treating a human being suffering from multiple sclerosis as a result of attack by T-cells of native tissue comprising mixing an anti-idiotype antibody specific for an antibody specific for the variable region of the a subunit of a human T-cell receptor, said region belonging to one of the V_{α} families 8-10, 12 or 16, or combinations thereof and being capable of inhibiting the activity of T-cells having said T-cell receptor or subunit thereof, and a pharmaceutically acceptable carrier and/or diluent.

5. A process for preparing a composition for use in a method for treating a human being suffering from multiple sclerosis as a result of attack by T-cells of native tissue comprising mixing an oligopeptide having substantially the same sequence as a variable region of the α subunit of a T-cell receptor, said region belonging to one of the human V_{α} families 8-10, 12 or 16, and being capable of inhibiting the activity of T-cells having said T-cell receptor or subunit thereof and a pharmaceutically acceptable carrier and/or diluent.

6. A process according to claim 6, wherein said family is the V_{α} 10 family.

## Patentansprüche (Patentansprüche für folgende(n) Vertragsstaat(en): AT, BE, CH, LI, DE, DK, FR, GB, IT, LU, NL, SE)

1. Zusammensetzung zur Verwendung in einem Verfahren zur Behandlung eines Menschen, der an multipler Sklerose als Ergebnis eines Angriffs durch T-Zellen auf natives Gewebe leidet, wobei die Zusammensetzung einen für eine variable Region der α- Untereinheit eines menschlichen T-Zell-Rezeptors spezifischen Antikörper umfasst, wobei die Region einer der V_{α}-Familien 8 bis 10, 12 oder 16 oder Kombinationen davon angehört, und der Antikörper die Aktivität von T-Zellen, auf denen der T-Zell-Rezeptor oder eine Untereinheit davon sich befindet, hemmen kann.

2. Zusammensetzung nach Anspruch 1, worin der Antikörper cytotoxisch ist.

3. Zusammensetzung nach Anspruch 2, worin die Familie die V_{α}-10-Familie ist.

4. Zusammensetzung zur Verwendung in einem Verfahren zur Behandlung eines Menschen, der an multipler Sklerose als Ergebnis eines Angriffs durch T-Zellen auf natives Gewebe leidet, wobei die Zusammensetzung einen Anti-Idiotyp-Antikörper umfasst, der für einen Antikörper spezifisch ist, der für eine variable Region der α-Untereinheit eines menschlichen T-Zell-Rezeptors spezifisch ist, wobei die Region einer der V_{α}-Familien 8 bis 10, 12 oder 16 oder Kombinationen davon angehört, und der Antikörper die Aktivität der T-Zellen, auf denen der T-Zell-Rezeptor oder eine Untereinheit davon ist, hemmen kann.

5. Zusammensetzung zur Verwendung in einem Verfahren zur Behandlung eines Menschen, der an multipler Sklerose als Ergebnis eines Angriffs durch T-Zellen auf natives Gewebe leitet, wobei die Zusammensetzung ein Oligopeptid mit im wesentlichen der gleichen Sequenz wie eine variable Region der α-Untereinheit eines T-Zell-Rezeptors umfasst, wobei die Region einer der menschlichen V_{α}-Familien 8 bis 10, 12 oder 16 angehört, und das Oligopeptid die Aktivität der T-Zellen, auf denen der T-Zell-Rezeptor oder eine Untereinheit davon ist, hemmen kann.

6. Zusammensetzung nach Anspruch 6, worin die Familie die V_{α}-10-Familie ist.

## Patentansprüche (Patentansprüche für folgende(n) Vertragsstaat(en): ES)

1. Zusammensetzung zur Verwendung in einem Verfahren zur Behandlung eines Menschen, der an multipler Sklerose als Ergebnis eines Angriffs durch T-Zellen auf natives Gewebe leidet, wobei die Zusammensetzung einen für eine variable Region der α-Untereinheit eines menschlichen T-Zell-Rezeptors spezifischen Antikörper umfasst, wobei die Region einer der V_{α}-Familien 8 bis 10, 12 oder 16 oder Kombinationen davon angehört, und der Antikörper die Aktivität von T-Zellen, auf denen der T-Zell-Rezeptor oder eine Untereinheit davon sich befindet, hemmen kann.

2. Zusammensetzung nach Anspruch 1, worin der Antikörper cytotoxisch ist.

3. Zusammensetzung nach Anspruch 2, worin die Familie die V_{α}-10-Familie ist.

4. Zusammensetzung zur Verwendung in einem Verfahren zur Behandlung eines Menschen, der an multipler Sklerose als Ergebnis eines Angriffs durch T-Zellen auf natives Gewebe leidet, wobei die Zusammensetzung einen Anti-Idiotyp-Antikörper umfasst, der für einen Antikörper spezifisch ist, der für eine variable Region der α-Untereinheit eines menschlichen T-Zell-Rezeptors spezifisch ist, wobei die Region einer der V_{α}-Familien 8 bis 10, 12 oder 16 oder Kombinationen davon angehört, und der Antikörper die Aktivität der T-Zellen, auf denen der T-Zell-Rezeptor oder eine Untereinheit davon ist, hemmen kann.

5. Zusammensetzung zur Verwendung in einem Verfahren zur Behandlung eines Menschen, der an multipler Sklerose als Ergebnis eines Angriffs durch T-Zellen auf natives Gewebe leidet, wobei die Zusammensetzung ein Oligopeptid mit im wesentlichen der gleichen Sequenz wie eine variable Region der α-Untereinheit eines T-Zell-Rezeptors umfasst, wobei die Region einer der menschlichen V_{α}-Familien 8 bis 10, 12 oder 16 angehört, und das Oligopeptid die Aktivität der T-Zellen, auf denen der T-Zell-Rezeptor oder eine Untereinheit davon ist, hemmen kann.

6. Zusammensetzung nach Anspruch 6, worin die Familie die V_{α}-Familie ist.

7. Verfahren zur Herstellung einer Zusammensetzung zur Verwendung in einem Verfahren zur Behandlung eines Menschen, der an multipler Sklerose als Ergebnis des Angriffs durch T-Zellen auf natives Gewebe leidet, wobei man einen Antikörper, der für eine variable Region der α-Untereinheit eines menschlichen T-Zell-Rezeptors spezifisch ist, wobei die Region einer der V_{α}-Familien 8 bis 10, 12 oder 16 oder Kombinationen davon angehört, und die Aktivität der T-Zellen, auf denen der T-Zell-Rezeptor oder eine Untereinheit davon ist, hemmen kann, und einen pharmazeutisch verträglichen Träger und/oder ein Verdünnungsmittel mischt.

8. Verfahren nach Anspruch 7, wobei der Antikörper cytotoxisch ist.

9. Verfahren nach Anspruch 8, wobei die Familie die V_{α}-10-Familie ist.

10. Verfahren zur Herstellung einer Zusammensetzung zur Verwendung in einem Verfahren zur Behandlung eines Menschen, der an multipler Sklerose als Ergebnis eines Angriffs durch T-Zellen auf natives Gewebe leidet, wobei man einen Anti-Idiotyp-spezifischen Antikörper, der für eine variable Region der α-Untereinheit eines menschlichen T-Zell-Rezeptors spezifisch ist, wobei die Region einer der V_{α}-Familien 8 bis 10, 12 oder 16 oder Kombinationen davon angehört, und die Aktivität der T-Zellen, auf denen der T-Zell-Rezeptor oder eine Untereinheit davon ist, hemmen kann, und einen pharmazeutisch verträglichen Träger und/oder ein Verdünnungsmittel mischt.

11. Verfahren zur Herstellung einer Zusammensetzung zur Verwendung in einem Verfahren zur Behandlung eines Menschen, der an multipler Sklerose als Ergebnis eines Angriffs durch T-Zellen auf natives Gewebe leidet, wobei man ein Oligopeptid mit im wesentlichen der gleichen Sequenz wie eine variable Region der α-Untereinheit eines T-Zell-Rezeptors, wobei die Region einer der menschlichen V_{α}-Familien 8 bis 10, 12 oder 16 angehört, das die Aktivität der T-Zellen, auf denen der T-Zell-Rezeptor oder eine Untereinheit davon ist, hemmen kann, und einen pharmazeutisch verträglichen Träger und/oder ein Verdünnungsmittel mischt.

12. Verfahren nach Anspruch 11, wobei die Familie die V_{α}-10-Familie ist.

## Patentansprüche (Patentansprüche für folgende(n) Vertragsstaat(en): GR)

1. Verfahren zur Herstellung einer Zusammensetzung zur Verwendung in einem Verfahren zur Behandlung eines Menschen, der an multipler Sklerose als Ergebnis des Angriffs durch T-Zellen auf natives Gewebe leidet, wobei man einen Antikörper, der für eine variable Region der α-Untereinheit eines menschlichen T-Zell-Rezeptors spezifisch ist, wobei die Region einer der V_{α}-Familien 8 bis 10, 12 oder 16 oder Kombinationen davon angehört, und die Aktivität der T-Zellen, auf denen der T-Zell-Rezeptor oder eine Untereinheit davon ist, hemmen kann, und einen pharmazeutisch verträglichen Träger und/oder ein Verdünnungsmittel mischt.

2. Verfahren nach Anspruch 1, wobei der Antikörper cytotoxisch ist.

3. Verfahren nach Anspruch 2, wobei die Familie die V_{α}-10-Familie ist.

4. Verfahren zur Herstellung einer Zusammensetzung zur Verwendung in einem Verfahren zur Behandlung eines Menschen, der an multipler Sklerose als Ergebnis eines Angriffs durch T-Zellen auf natives Gewebe leidet, wobei man einen Anti-Idiotyp Antikörper, der für einen Antikörper, der für eine variable Region der α-Untereinheit eines menschlichen T-Zell-Rezeptors spezifisch ist, spezifisch ist, wobei die Region einer der V_{α}-Familien 8 bis 10, 12 oder 16 oder Kombinationen davon angehört, und die Aktivität der T-Zellen, auf denen der T-Zell-Rezeptor oder eine Untereinheit davon ist, hemmen kann, und einen pharmazeutisch verträglichen Träger und/oder ein Verdünnungsmittel mischt.

5. Verfahren zur Herstellung einer Zusammensetzung zur Verwendung in einen Verfahren zur Behandlung eines Menschen, der an multipler Sklerose als Ergebnis eines Angriffs durch T-Zellen auf natives Gewebe leidet, wobei man ein Oligopeptid mit im wesentlichen der gleichen Sequenz wie eine variable Region der α-Untereinheit eines T-Zell-Rezeptors, wobei die Region einer der menschlichen V_{α}-Familien 8 bis 10, 12 oder 16 angehört, und das die Aktivität der T-Zellen, auf denen der T-Zell-Rezeptor oder eine Untereinheit davon ist, hemmen kann, und einen pharmazeutisch verträglichen Träger und/oder ein Verdünnungsmittel mischt.

6. Verfahren nach Anspruch 6, wobei die Familie die V_{α}-10-Familie ist.

## Revendications (Revendications pour l'(les) Etat(s) contractant(s) suivant(s): AT, BE, CH, LI, DE, DK, FR, GB, IT, LU, NL, SE)

1. Composition destinée à être utilisée dans une méthode de traitement d'un patient humain souffrant de sclérose en plaques en résultat d'une attaque par les lymphocytes T d'un tissu naturel, ladite composition comprenant un anticorps spécifique d'une région variable de la sous-unité α d'un récepteur de lymphocytes T humains, ladite région appartenant à une des catégories V_{α} 8-10, 12 et 16, ou de leurs associations et étant capable d'inhiber l'activité de lymphocytes T comportant ledit récepteur de lymphocytes T ou sa sous-unité.

2. Composition suivant la revendication 1, dans laquelle l'anticorps est cytotoxique.

3. Composition suivant la revendication 2, dans laquelle la catégorie est la catégorie V_{α} 10.

4. Composition destinée à être utilisée dans une méthode de traitement d'un patient humain souffrant de sclérose en plaques en résultat d'une attaque par des lymphocytes T d'un tissu naturel, ladite composition comprenant un anticorps anti-idiotype spécifique d'un anticorps spécifique d'une région variable de la sous-unité α d'un récepteur de lymphocytes T humains, ladite région appartenant à une des catégories V_{α} 8-10, 12 et 16 ou leurs associations et étant capable d'inhiber l'activité de lymphocytes T comportant ledit récepteur de lymphocytes T ou sa sous-unité.

5. Composition destinée à être utilisée dans une méthode de traitement d'un patient humain souffrant de sclérose en plaques en résultat d'une attaque par des lymphocytes T d'un tissu naturel, ladite composition comprenant :
un oligopeptide ayant pratiquement la même séquence qu'une région variable de la sous-unité α d'un récepteur de lymphocytes T, ladite région appartenant à une des catégories humaines V_{α} 8-10, 12 et 16 ;
et étant capable d'inhiber l'activité de lymphocytes T comportant ledit récepteur de lymphocytes T ou sa sous-unité.

6. Composition suivant la revendication 5, dans laquelle la catégorie est la catégorie V_{α} 10.

## Revendications (Revendications pour l'(les) Etat(s) contractant(s) suivant(s): ES)

1. Composition destinée à être utilisée dans une méthode de traitement d'un patient humain souffrant de sclérose en plaques en résultat d'une attaque par des lymphocytes T d'un tissu naturel, ladite composition comprenant un anticorps spécifique d'une région variable de la sous-unité α d'un récepteur de lymphocytes T humains, ladite région appartenant à une des catégories V_{α} 8-10, 12 et 16, ou leurs associations et étant capable d'inhiber l'activité de lymphocytes T comportant ledit récepteur de lymphocytes T ou sa sous-unité.

2. Composition suivant la revendication 1, dans laquelle l'anticorps est cytotoxique.

3. Composition suivant la revendication 2, dans laquelle la catégorie est la catégorie V_{α} 10.

4. Composition destinée à être utilisée dans une méthode de traitement d'un patient humain souffrant de sclérose en plaques en résultat d'une attaque par des lymphocytes T d'un tissu naturel, ladite composition comprenant un anticorps anti-idiotype spécifique d'un anticorps spécifique d'une région variable de la sous-unité α d'un récepteur de lymphocytes T humains, ladite région appartenant à une des catégories V_{α} 8-10, 12 et 16 ou leurs associations et étant capable d'inhiber l'activité de lymphocytes T comportant ledit récepteur de lymphocytes T ou sa sous-unité.

5. Composition destinée à être utilisée dans une méthode de traitement d'un patient humain souffrant de sclérose en plaques en résultat d'une attaque par des lymphocytes T d'un tissu naturel, ladite composition comprenant :
un oligopeptide ayant pratiquement la même séquence qu'une région variable de la sous-unité α d'un récepteur de lymphocytes T, ladite région appartenant à une des catégories humaines V_{α} 8-10, 12 et 16 ;
et étant capable d'inhiber l'activité de lymphocytes T comportant ledit récepteur de lymphocytes T ou sa sous-unité.

6. Composition suivant la revendication 5, dans laquelle la catégorie est la catégorie V_{α} 10.

7. Procédé pour la préparation d'une composition destinée à être utilisée dans une méthode de traitement d'un patient humain souffrant de sclérose en plaques en résultat d'une attaque par les lymphocytes T d'un tissu naturel, comprenant le mélange d'un anti-corps spécifique d'une région variable de la sous-unité α d'un récepteur de lymphocytes T humains, ladite région appartenant à une des catégories V_{α} 8-10, 12 ou 16, ou leurs associations, et étant capable d'inhiber l'activité de lymphocytes T comportant ledit récepteur de lymphocytes T ou sa sous-unité, et un support et/ou diluant pharmaceutiquement acceptable.

8. Procédé suivant la revendication 7, dans lequel l'anti-corps est cytotoxique.

9. Procédé suivant la revendication 8, dans lequel la catégorie est la catégorie V_{α} 10.

10. Procédé pour la préparation d'une composition destinée à être utilisée dans une méthode de traitement d'un patient humain souffrant de sclérose en plaques en résultat de l'attaque par les lymphocytes T d'un tissu naturel, comprenant le mélange d'un anti-corps anti-idiotype spécifique d'une région variable de la sous-unité α d'un récepteur de lymphocytes T humains, ladite région appartenant à une des catégories V_{α} 8-10, 12 ou 16 ou leurs associations et étant capable d'inhiber l'activité de lymphocytes T comportant ledit récepteur de lymphocytes T ou sa sous-unité, et un support et/ou diluant pharmaceutiquement acceptable.

11. Procédé pour la préparation d'une composition destinée à être utilisée dans une méthode de traitement d'un patient humain souffrant de sclérose en plaques en résultat d'une attaque par des lymphocytes T d'un tissu naturel, comprenant le mélange d'un oligopeptide ayant pratiquement la même séquence qu'une région variable de la sous-unité α d'un récepteur de lymphocytes T, ladite région appartenant à une des catégories V_{α} humaines 8-10, 12 ou 16 et étant capable d'inhiber l'activité de lymphocytes T comportant ledit récepteur de lymphocytes T ou sa sous-unité, et un support et/ou diluant pharmaceutiquement acceptable.

12. Procédé suivant la revendication 11, dans lequel la catégorie est la catégorie V_{α} 10.

## Revendications (Revendications pour l'(les) Etat(s) contractant(s) suivant(s): GR)

1. Procédé de préparation d'une composition destinée à être utilisée dans une méthode de traitement d'un patient humain souffrant de sclérose en plaques en résultat d'une attaque par des lymphocytes T d'un tissu naturel, comprenant le mélange d'un anticorps spécifique de la région variable de la sous-unité α d'un récepteur de lymphocytes T humains, ladite région appartenant à une des catégories V_{α} 8-10, 12 et 16 ou leurs associations et étant capable d'inhiber l'activité de lymphocytes T comportant ledit récepteur de lymphocytes T ou sa sous-unité, et d'un support et/ou d'un diluant pharmaceutiquement acceptables.

2. Procédé suivant la revendication 1, dans lequel l'anticorps est un anticorps cytotoxique.

3. Procédé suivant la revendication 2, dans lequel la catégorie est la catégorie V_{α} 10.

4. Procédé de préparation d'une composition destinée à être utilisée dans une méthode de traitement d'un patient humain souffrant de sclérose en plaques en résultat d'une attaque par des lymphocytes T d'un tissu naturel, comprenant le mélange d'un anticorps anti-idiotype spécifique d'un anticorps spécifique de la région variable de la sous-unité α d'un récepteur de lymphocytes T humains, ladite région appartenant à une des catégories V_{α} 8-10, 12 et 16 ou leurs associations et étant capable d'inhiber l'activité de lymphocytes T comportant ledit récepteur de lymphocytes T ou sa sous-unité, et d'un support et/ou d'un diluant pharmaceutiquement acceptables.

5. Procédé de préparation d'une composition destinée à être utilisée dans une méthode de traitement d'un patient humain souffrant de sclérose en plaques en résultat d'une attaque par des lymphocytes T d'un tissu naturel, comprenant le mélange d'un oligopeptide ayant pratiquement la même séquence qu'une région variable de la sous-unité α d'un récepteur de lymphocytes T, ladite région appartenant à une des catégories humaines V_{α} 8-10, 12 et 16 et étant capable d'inhiber l'activité de lymphocytes T comportant ledit récepteur de lymphocytes T ou sa sous-unité, et d'un support et/ou d'un diluant pharmaceutiquement acceptables.

6. Procédé suivant la revendication 5, dans lequel la catégorie est la catégorie V_{α} 10.
